# EUROPEAN PATENT SPECIFICATION

(11) **EP 1 646 369 B1**
(45) Date of publication and mention of the grant of the patent: **10.04.2019**
(21) Application number: 04763093.4
(22) Date of filing: 05.07.2004
(51) Int. Cl.: A61K 9/16, A61K 9/20, A61K 31/551, A61P 31/18

(54) **SAQUINAVIR MESYLATE ORAL DOSAGE FORM**
SAQUINAVIR-MESYLAT ORALE DOSIERFORM
FORME DOSIFIEE ORALE DE MESYLATE DE SAQUINAVIR

(30) Priority: 11.07.2003 US 486600 P; 05.05.2004 US 568204 P
(43) Date of publication of application: 19.04.2006
(73) Proprietor: F. Hoffmann-La Roche AG, 4070 Basel (CH)
(72) Inventor: ALBANO, Antonio, A., Clifton, NJ 07013 (US); INFELD, Martin, Howard, Upper Montclair, NJ 07043 (US); PHUAPRADIT, Wantanee, Montville, NJ 07045 (US); SHAH, Navnit-Hargovindas, Clifton, NJ 07012 (US); ZHANG, Lin, Nutley, New Jersey 07110 (US)
(74) Representative: Rauber, Beat
(86) International application number: PCT/EP2004/007309
(87) International publication number: WO 2005/004836

(56) References cited:
- WO-A-99/12527
- WO-A-03/005944
- US-A- 6 039 975
- Anonymous: "INVIRASE (saquinavir mesylate) CAPSULES", INTERNET ARTICLE, 1 January 2001 (2001-01-01), XP55039675, Retrieved from the Internet: URL:http://www.accessdata.fda.gov/drugsatf da_docs/label/2002/20628s16lbl.pdf [retrieved on 2012-10-01]
- Anonymous: "Invirase (R) capsule Mix (200 mg)", INTERNET ARTICLE, 1 January 2001 (2001-01-01), XP55039673, Retrieved from the Internet: URL:http://www.gene.com/gene/products/info rmation/msds/invirase/MSDS_INVIRASE_CapMix 200mg.pdf [retrieved on 2012-10-01]

## Description

Saquinavir mesylate is one of several protease inhibitors used to limit viral replication and improve immune function in HIV-infected individuals. Saquinavir mesylate is commercially available as a 200 mg capsule (calculated as Saquinavir free base). It is sold under the name INVIRASE® by Hoffmann-La Roche, Inc., and is indicated for use in combination with an antiretroviral nucleoside analogue for the treatment of advanced human immunodeficiency virus (HIV) infection in select patients.

Saquinavir mesylate is a white to off-white, very fine crystalline powder having a molecular weight of 766.96. The molecular weight of the free base is 670.86. The drug is highly hydrophobic. INVIRASE® (Saquinavir Mesylate) 200 mg capsules have low oral bioavailability, which is thought to be due to its incomplete absorption and extensive first pass metabolism. [Physician's Desk Reference, 57th Ed. (2003).] Saquinavir mesylate has very low aqueous solubility (i.e. 2.2 mg/mL in water, 0.08 mg/mL in simulated gastric fluid and is practically insoluble in simulated intestinal fluid at 25°C). In addition, the drug exhibits pH-dependent solubility characteristics, having limited solubility in simulated gastric fluid while being practically insoluble in simulated intestinal fluid. Absolute bioavailability averaged 4% (CV 73%, range: 1-9%) in 8 healthy volunteers who received a single 600 mg dose (3 x 200 mg capsules) of saquinavir following a high fat breakfast. Saquinavir 24-hour AUC and Cmax (n=6) following the administration of a higher calorie meal were on average two times higher than after a lower calorie meal. The effect of food has been shown to persist for up to 2 hours. To attempt to minimize inter-subject variability and the food effect of saquinavir mesylate seen in man, an oral solid dosage form having a uniform and rapid dissolution profile of saquinavir mesylate would be desirable.

Particle size reduction is one means in which to minimize inter-subject variability and improving bioavailability. Micronization is an approach used to reduce particle size. Upon micronization, saquinavir mesylate has a tendency to agglomerate, however, thus reducing the surface area of its primary particles in contact with the dissolution medium. Micronized saquinavir mesylate exhibits a slow dissolution rate.

For adult patients, the recommended dose of INVIRASE® in combination with a nucleoside analogue is 3 x 200 mg capsules three times daily within 2 to 4 hours after food. In view of this dosage regime, patient compliance is a real concern. Treatment success could be improved by encouraging better adherence, as for example, by reducing the number of dosage units that must be taken per day. A unit dosage form containing a higher amount of saquinavir would thus be useful. The problem of drug agglomeration worsens, however, with increased drug loading of micronized saquinavir mesylate.

The present invention provides a solid unit oral pharmaceutical dosage form of saquinavir mesylate comprising from 60% to 80% micronized saquinavir mesylate based on the mesylate salt, from 4% to 8% of the pharmaceutically acceptable water soluble binder polyvinylpyrrolidone, 3% to 10% of a pharmaceutically acceptable disintegrant selected from croscarmellose sodium and crospovidone, and 3% to 10% of the pharmaceutically acceptable carrier lactose monohydrate, wherein each percentage is of the kernel weight of the pharmaceutical dosage form. (The 60% to 80% micronized saquinavir mesylate corresponds to an amount of from about 200 mg to about 800 mg saquinavir mesylate calculated as saquinavir free base.)

In a preferred embodiment the saquinavir mesylate is from about 70% to 75% of the kernel weight of the solid unit oral pharmaceutical dosage form.

The present invention provides a solid unit oral pharmaceutical dosage form of saquinavir mesylate comprising micronized saquinavir mesylate in an amount of from about 250 mg to about 800 mg calculated as free base, a pharmaceutically acceptable binder, a pharmaceutically acceptable disintegrant, and a pharmaceutically acceptable water soluble carrier.

### Brief description of the drawings:

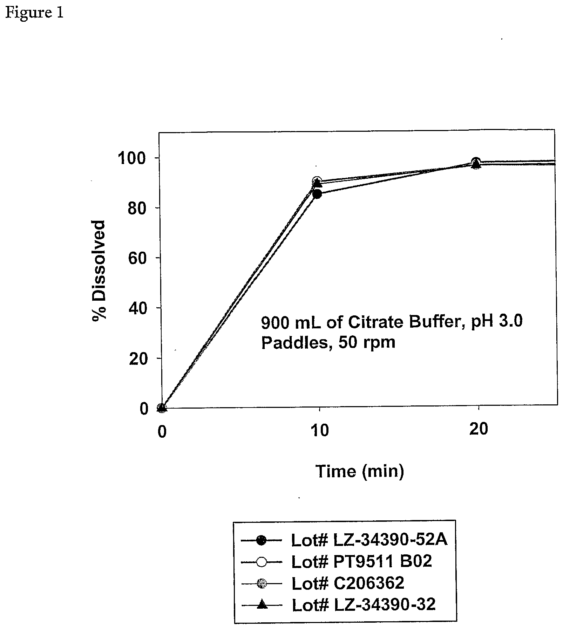

Figure 1 presents dissolution profiles of the invented formulation in a tablet dosage form, Example 1 (500 mg as free base), indicating lot-to-lot reproducibility

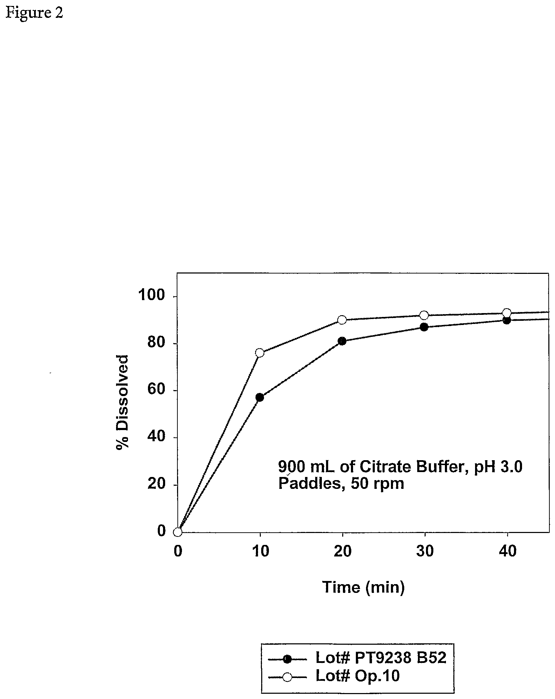

Figure 2 presents dissolution profiles of the current market formulation in a capsule dosage form, Comparative Example 2 (200 mg as free base), indicating lot-to-lot variability.

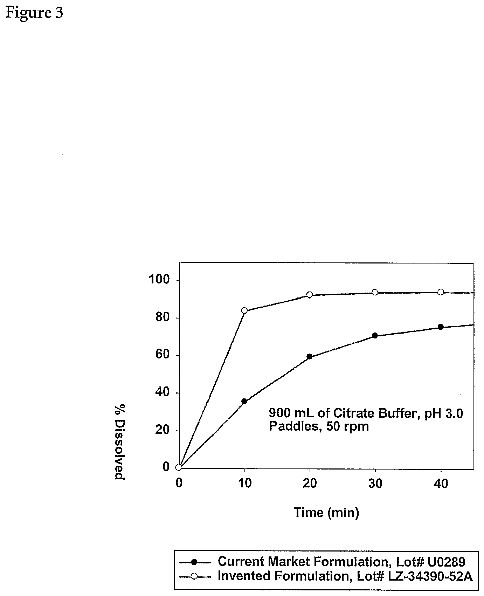

Figure 3 presents dissolution profiles of the invented formulation in a tablet dosage form (Example 1) compared to the current market formulation in a capsule dosage form (Comparative Example 2) at a dose of 1000 mg saquinavir as free base.

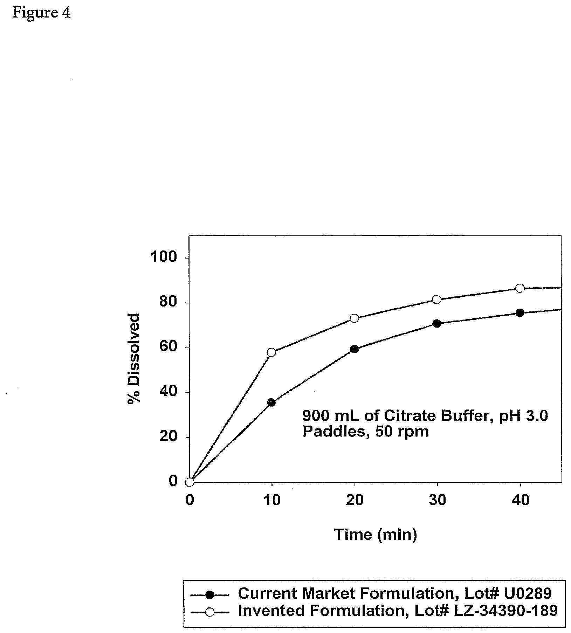

Figure 4 presents dissolution profiles of the invented formulation in a capsule dosage form (Example 3) compared to the current market formulation in a capsule dosage form (Comparative Example 2) at a dose of 1000 mg saquinavir as free base

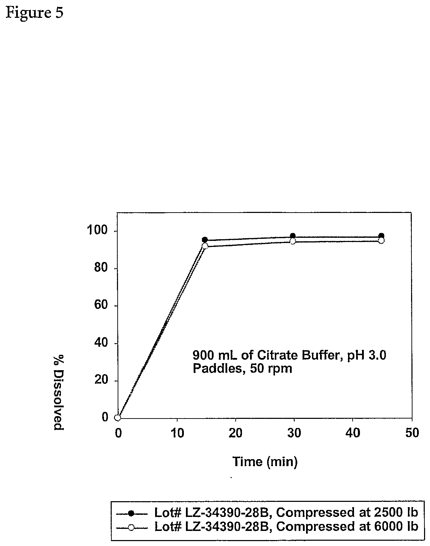

Figure 5 presents dissolution profiles of the invented formulation in a tablet dosage form, Example 1 (500 mg as saquinavir free base), indicating a rapid and highly reproducible dissolution profile regardless of compression force applied

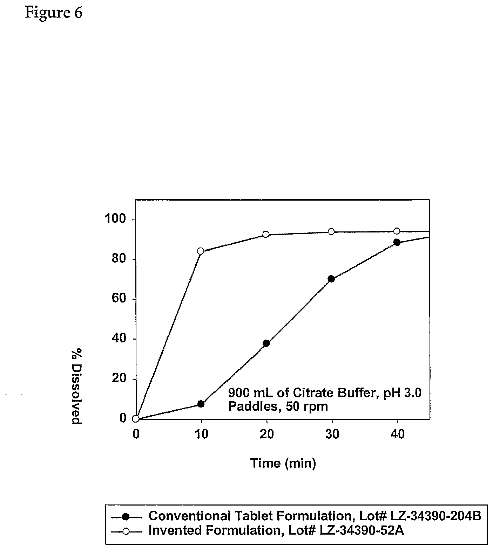

Figure 6 presents dissolution profiles of the invented formulation in a tablet dosage form (Example 1) compared to a conventional tablet formulation (Comparative Example 4) at a dose of 1000 mg saquinavir as free base.

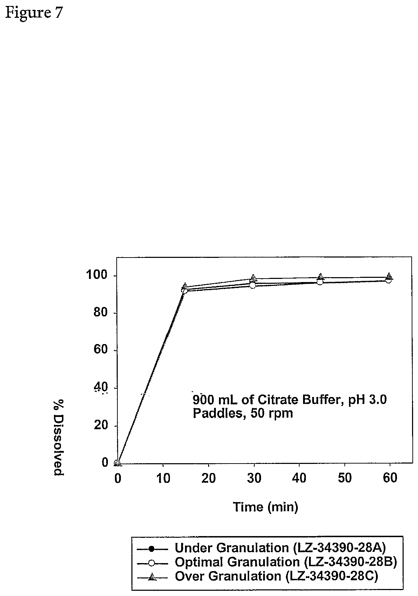

Figure 7 presents dissolution profiles of the invented formulation in a tablet dosage form, Example 1 (500 mg as free base), indicating a rapid and highly reproducible dissolution profile regardless of the granulation end point.

The pharmaceutical dosage form of micronized saquinavir mesylate in accordance with the present invention provides a rapid and highly reproducible dissolution profile. The saquinavir mesylate dosage form of the present invention may be used to treat HIV-infected individuals. Coadministration with another antiretroviral drug, for example, ritonavir, is contemplated.

The present invention provides a solid unit oral pharmaceutical dosage form of saquinavir mesylate comprising micronized saquinavir mesylate in an amount of from about 200 mg to about 800 mg, preferably 200 to 700 mg, more preferably 250 to 700 mg, even more preferably 500 mg calculated as saquinavir free base.

The solid unit oral pharmaceutical dosage form of saquinavir mesylate further comprises lactose monohydrate as pharmaceutically acceptable carrier, which is present from about 3% to about 10%, preferably from about 4% to 6% by weight of the kernel. The water soluble carrier expediently has a particle size from about 30 micrometers to 200 micrometers. Preferably lactose monohydrate has a particle size of from about 100 micrometers to 150 micrometers.

A pharmaceutically acceptable water soluble binder is present from about 4% to 8%, preferably from about 4% to 6% by weight of the kernel. The water soluble binder is a polyvinylpyrrolidone. Well suited are polyvinylpyrrolidones which are traded under the tradename Povidone such as the Povidone K30.

A pharmaceutically acceptable disintegrant is present from about 3% to 10%, preferably from about 4% to 8% by weight of the kernel. The pharmaceutically acceptable disintegrants are selected from croscarmellose sodium and crospovidone.

A granulation of the saquinavir mesylate, the carrier, the binder, and at least a portion of the disintegrant is prepared. This granulation consists of various particle sizes of agglomerates of saquinavir mesylate, carrier, binder and disintegrant, and the resulting powder is free-flowing with advantageous compaction and wetting properties. When the unit dosage is a tablet, the tablet is prepared in part from these agglomerates. When the tablet is exposed to gastrointestinal fluids, it disintegrates and releases micronized saquinavir mesylate for rapid dissolution.

After preparing the granulation, microcrystalline cellulose (MCC) may be added as an extra-granular component to enhance mechanical strength of the produced tablets. MCC is expediently present from about 5 to 20%, preferably from about 5% to 15% by weight of the kernel.

A lubricant such as magnesium stearate maybe added as an extra-granular component, from about 0.5 to 1.2% by weight of the kernel.

The present invention also provides a process for preparing a solid unit oral pharmaceutical dosage form of saquinavir mesylate. The process involves micro-granulation of the drug with a disintegrant and a hydrophilic binder and carrier. The dosage form thus produced retains saquinavir mesylate in crystalline form.

The invented formulation, in either a tablet or capsule dosage form, exhibits a relatively faster and much more reproducible dissolution profile compared to the profile of the current market capsule formulation. Moreover, the oral dosage form disclosed herein provides a rapid and highly reproducible dissolution profile, irrespective of compression force and granulation end point. The dosage form of the present invention advantageously has a weight of from about 400 mg to about 1.5 g.

The solid unit oral pharmaceutical dosage form of the present invention contains a kernel and a kernel containing portion. The kernel comprises the saquinavir mesylate, binder, disintegrant, and carrier, in accordance with the present invention. The kernel optionally includes one or more pharmaceutically acceptable excipients, for example, lactose monohydrate. The kernel is preferably comprised of an admixture of a granulation and excipients added to the granulation ("extra-granulation"). The kernel containing portion may be, for example, a tablet film coating, or a capsule or caplet coating.

The saquinavir mesylate used in the present invention is micronized to small particle size. Micronized saquinavir mesylate is typically saquinavir mesylate having particles ranging from about 1 to about 20 micrometers. In preparing the solid unit oral pharmaceutical dosage form, micronized saquinavir mesylate calculated based on the mesylate salt is used in an amount such that it is about 60% to about 80% of the kernel weight. This corresponds to an amount of micronized saquinavir mesylate calculated as saquinavir free base of from about 200 mg to about 800 mg.

Pharmaceutically acceptable excipients which may optionally be used in the present invention include types of excipients other than those recited. Thus, the percentage of binder and disintegrant in the kernel, for example, remain as indicated. Pharmaceutically acceptable excipients that may optionally be used include microcrystalline cellulose and lubricants.

Lubricants include, for example, magnesium stearate and talc. Magnesium stearate is preferred. A lubricant can be used as an extra-granulation ingredient of the kernel. A lubricant is preferably present from 0.5% to 1.2% by weight of the kernel.

The excipient added to the milled, dried granulation can be selected from the group of lubricants, disintegrants and diluents. The pharmaceutical excipient may be, for example, microcrystalline cellulose, corn starch, magnesium stearate, etc. For preparing tablets, the solid dosage form can be processed into a solid unit oral dosage form by granulating, milling, blending, lubricating, compressing (tabletting), and, typically, aqueous film coating.

The pharmaceutical dosage form of the present invention is prepared by micro-granulating micronized saquinavir mesylate with the disintegrant and the hydrophilic binder and carrier, and milling. Preferably, the granulation is blended with lubricant, tabletted and aqueous-based film coated. During the micro-granulation process, micronized saquinavir mesylate is deagglomerated and wetted by the hydrophilic carrier and binder, thereby maximizing the surface area of its primary particles in contact with the dissolution medium.

A method for preparing a solid unit oral pharmaceutical dosage form of micronized saquinavir mesylate is provided, comprising spraying a solution of water soluble binder on an admixture of from about 200 mg to about 800 mg micronized saquinavir mesylate calculated as free base, a pharmaceutically acceptable disintegrant, and a pharmaceutically acceptable water soluble carrier, to achieve a uniform granulation of the present invention.

Preferably, the carrier is lactose monohydrate, and the
disintegrant is croscarmellose sodium.

Preferably, a portion of the disintegrant is included in the granulation, and the remaining portion of the disintegrant is added as an extra-granular component, and blended. The ratio of disintegrant in the granulation to disintegrant in the extra-granulation is from about 3:1 to about 1:1. Preferably, the ratio is from about 2.5:1 to about 1.5:1. More preferably, the ratio is about 2:1.

Preferably, microcrystalline cellulose is added as an extra-granular component and blended with the granulation to enhance mechanical strength of the resulting tablets. Microcrystalline cellulose is present from about 5% to about 20% by weight of the kernel,
preferably from about 5% to about 15%.

Preferably, a lubricant such as magnesium stearate, is added externally to the granulation to provide adequate lubricity to tablet punch tooling during compression. The lubricant is present from about 0.5% to about 1.2% by weight of the kernel.

In an embodiment of the present invention, tablets are prepared as follows:
a) Blend micronized saquinavir mesylate in an amount of from about 200 mg to about 800 mg (calculated as free base) per unit dosage with a hydrophilic carrier and disintegrant in a high shear granulator.
b) Spray or slowly add an aqueous binder solution in water to the powder mix from step (a) while mixing to achieve the optimal granulation end point.
c) De-lump the wet granulation from step (b) into a uniform granulation.
d) Dry the wet granulation from step (c) in a forced-air oven set at 40°-50°C or in a fluid bed dryer with inlet air temperature of 50°-60°C until the moisture content of the granulation is in the range of 1.5-2%.
e) Mill the dried granulation from step (d).
f) Blend the milled granulation from step (e) with other suitable tablet diluents, such as microcrystalline cellulose, and disintegrant.
g) Lubricate the blend from step (f) with a suitable lubricant, such as magnesium stearate.
h) Compress the final blend from step (g) on a tablet press.
i) Aqueous film coat the tablet from step (h).

### Examples

In the following Examples, the optimal granulation end point was determined by visual inspection as the point at which the granulation had no further detectable change in particle size.

### Example 1:

**Table 1: 500 mg Saquinavir Mesylate Tablet (Invented Formulation)**

| Ingredients | mg/tablet |
|---|---|
| Micronized Saquinavir Mesylate | 571.50* |
| Lactose Monohydrate | 38.50 |
| Croscarmellose Sodium | 45.00 |
| Povidone K30 | 40.00 |
| Microcrystalline Cellulose (Avicel PH 101) | 95.00 |
| Magnesium Stearate | 10.00 |
| KERNEL WEIGHT | 800.00 |
| Hydroxypropyl Methylcellulose 2910 (6 cps) | 7.01 |
| Titanium Dioxide | 4.32 |
| Talcum | 4.32 |
| Iron Oxide Yellow | 0.57 |
| Iron Oxide Red | 0.08 |
| Ethylcellulose Dispersion (solids) | 2.32 |
| Triacetin | 1.38 |
| TOTAL WEIGHT | 820.00 |

| | |
|---|---|
| * Equivalent to 500 mg as free base | |

### I. PREPARATION OF KERNELS

A. Micronized saquinavir mesylate, lactose monohydrate and a portion of croscarmellose sodium (approximately 66.7% of total amount of croscarmellose sodium) were mixed in a high shear granulator for 5 minutes using impeller at low speed and agitator at low speed.
B. Preparation of 20% w/w Povidone K30 Solution: In a stainless steel container, Povidone K30 was slowly added to Purified Water (160 mg/tablet) and mixed using a propeller mixer. Mixing was continued until Povidone K30 was completely dissolved.
C. (1). The powder mix from Step A was granulated by spraying the 20% w/w Povidone K30 Solution from Step B onto the powder mix in the high shear granulator and continually mixed using impeller at low speed and agitator at low speed for over 8 -10 minutes.
C. (2). Additional Purified Water (approximately 180 mg/tablet) was sprayed onto the powder mix from Step C(1) which was continually mixed using impeller at low speed and agitator at low speed for over 8 - 10 minutes. Additional kneading of the granulation was performed to achieve an optimal granulation end point. The wet granulation was discharged with impeller at low speed and agitator at low speed into a polyethylene-lined container.
D. The wet granulation was delumped by passing through a Co-mil equipped with a 19.05-mm round opening screen (#750Q) at 1350 rpm or through a Frewitt rotating sieve equipped with a 10-mm round opening screen at 1000 -2000 rpm.
E. The delumped wet granulation from Step D was dried in a fluid bed dryer with inlet air temperature set at 65°±10°C until the moisture content of the granulation, determined by loss on drying using an Omnimark Moisture Analyzer set at 90°C, was less than 1.8%.
F. The dried granulation from Step E was milled through a Co-mil equipped with a 1.27-mm round, grated opening screen (#050G) at 4500 rpm or through a Frewitt hammer mill equipped with a 2.0-mm round screen using knives forward at 3170 rpm.
G. The milled granulation from Step F, Avicel PH 101 and the remainder of croscarmellose sodium (approximately 33.3% of total croscarmellose sodium) were mixed in a PK Blender or equivalent for 10 minutes.
H. Approximately 50% of the granulation from the PK Blender in Step G was removed.
I. Magnesium Stearate (passed through a #30 mesh stainless steel screen) was added to the PK Blender from Step H. The removed granulation from Step H was placed back into the PK Blender and mixed for 5 minutes.
J. The granulation from Step I was compressed using the following specifications:

| | |
|---|---|
| Punch Size: | Oval-shaped, 8.74 mm x 18.75 mm, standard concave |
| Tablet Weight: | 800 mg (760 - 840 mg) |
| Tablet Hardness: | 30 SCU (25 - 35 SCU) or 210 N (175 -245 N) |

### II. APPLICATION OF THE FILM COAT

### A. Preparation of the Film Coating Suspension

In a stainless steel container, Triacetin and Aquacoat ECD-30 were dispersed in Purified Water using a propeller mixer and mixed for 45 minutes.

A powder mixture of hydroxypropyl methylcellulose 2910 (6 cps), talc, titanium dioxide, yellow iron oxide and red iron oxide was added to the dispersion, which was mixed gently to avoid air entrapment. Mixing was continued for another 60 minutes or until a uniform suspension was obtained.

### B. Application of the Film Coat

The kernels from Step J of Section I (PREPARATION OF KERNELS) were placed into a perforated coating pan. The inlet temperature was slowly increased to 60°±10°C to warm the kernels, with intermittent jogging, until the outlet temperature reached 40°±5°C.

With the inlet air temperature at 60°±10°C, the pan speed was increased to provide sufficient rotation of the kernels inside the pan. The kernels were sprayed with the Film Coating Suspension from Section IIA above and stirred continuously using an air spray system. The product temperature was maintained at 45°±5°C. 20 mg of the film coat (range 17-23 mg) was applied on a dry basis per tablet.

The inlet air temperature was reduced to 50°±5°C and the pan speed to 4 ± 2 rpm. Drying of the coated tablets was continued for 2-4 minutes.

The inlet air temperature was reduced to 40°±5°C and the coated tablets were dried by jogging until the moisture content of the tablets, determined by loss on drying using an Omnimark Moisture Analyzer set at 90°C, was less than 2.0%. The heat was turned off and the tablets cooled to room temperature by occasional jogging.

### Comparative Example 2:

**Table 2: 200 mg Saquinavir Mesylate Capsule (Market Formulation)**

| Ingredients | mg/ capsule |
|---|---|
| Micronized Saquinavir Mesylate | 228.70* |
| Sodium Starch Glycolate | 16.00 |
| Lactose Anhydrous | 63.30 |
| Microcrystalline Cellulose (Avicel PH 102) | 60.00 |
| Povidone K30 | 8.00 |
| Magnesium Stearate | 4.00 |
| Talcum | 28.00 |
| FILL WEIGHT | 408.00 |
| Capsule Shell (Size #0) | 96.00 |
| TOTAL WEIGHT | 504.00 |

| | |
|---|---|
| *Equivalent to 200 mg as free base | |

A. Micronized saquinavir mesylate, lactose anhydrous, microcrystalline cellulose and a portion of sodium starch glycolate (56.25% of total amount of sodium starch glycolate) were mixed in a high shear granulator.
B. Povidone K30 Solution was added to the powder mix in the high shear granulator (Step A) and continually mixed, to granulate the powder mix.
C. Additional Purified Water was added to the powder mix from Step B, which was continually mixed until an optimal granulation end point was obtained. The wet granulation was discharged into a polyethylene-lined container.
D. The wet granulation from Step C was delumped through a mill.
E. The delumped wet granulation from Step D was dried in a fluid bed dryer with inlet air temperature set at 65°±10°C until the moisture content of the granulation, determined by loss on drying using an Omnimark Moisture Analyzer set at 90°C, was less than 1.8%.
F. The dried granulation from Step E was passed through a mill.
G. The milled granulation from Step F was mixed with a portion of sodium starch glycolate (43.75% of total amount of sodium glycolate, talc and magnesium stearate) in a blender.
H. The final blend from Step G was encapsulated in a capsule (#0) at a target fill weight of 408 mg using a capsule filling machine.

### Example 3

**Table 3: 200 mg Saquinavir Mesylate Capsule (Invented Formulation)**

| Ingredients | Mg/capsule |
|---|---|
| Micronized Saquinavir Mesylate | 228.70* |
| Lactose Monohydrate | 15.30 |
| Sodium Croscarmellose | 18.00 |
| Povidone K30 | 16.00 |
| Microcrystalline Cellulose (Avicel PH 101) | 38.00 |
| Magnesium Stearate | 4.00 |
| FILL WEIGHT | 320.00 |
| Capsule Shell (Size #0) | 96.00 |
| TOTAL WEIGHT | 416.00 |

| | |
|---|---|
| * Equivalent to 200 mg as free base | |

For Steps A to I, follow the same procedure specified in Example 1.

Step J. The final blend from Step I was encapsulated in a capsule (#0) at a target fill weight of 320 mg using a capsule filling machine.

### Comparative Example 4

**Table 4: 500 mg Saquinavir Mesylate Tablet Produced by Prior Methodology**

| Ingredients | Mg/tablet |
|---|---|
| Micronized Saquinavir Mesylate | 571.50* |
| Povidone K30 | 20.00 |
| Lactose Anhydrous | 158.25 |
| Microcrystalline Cellulose (Avicel PH 102) | 150.00 |
| Sodium Starch Glycolate | 40.00 |
| Talcum | 70.00 |
| Magnesium Stearate | 10.00 |
| TOTAL TABLET WEIGHT | 1019.75 |

| | |
|---|---|
| * Equivalent to 500 mg free base | |

For Steps A to G, follow the same procedures specified in Example 2.

Step H. The granulation from Step G was compressed using the following specifications:

| | |
|---|---|
| Punch Size: | Oval-shaped, 9.28 mm x 20.02 mm, standard concave |
| Tablet Weight: | 1200 mg (1140 - 1260 mg) |
| Tablet Hardness: | 25 - 35 SCU or 175 -245 N |

### Dissolution Testing:

Oral dosage forms containing saquinavir mesylate (Examples 1-4) were evaluated for dissolution in 900 mL of citrate buffer, pH 3.0, equilibrated at 37°±0.5°C using a paddle method (USP Apparatus 2) at 50 rpm. Sample aliquots were taken at different time intervals and analyzed by UV spectrophotometry.

## Claims

1. A solid unit oral pharmaceutical dosage form of saquinavir mesylate comprising from 60% to 80% micronized saquinavir mesylate based on the mesylate salt, from 4% to 8% of polyvinylpyrrolidone, 3% to 10% of a pharmaceutically acceptable disintegrant selected from croscarmellose sodium and crospovidone, and 3% to 10% of lactose monohydrate, wherein each percentage is of the kernel weight of the pharmaceutical dosage form.

2. The dosage form according to claim 1, wherein the saquinavir mesylate is from 70% to 75% of the kernel weight of the pharmaceutical dosage form.

3. The dosage form according to claim 1 or 2, wherein the saquinavir mesylate in the pharmaceutical dosage form is in an amount of from 200 mg to 700 mg calculated as saquinavir free base.

4. The dosage form according to claims 1 to 3, wherein the saquinavir mesylate in the pharmaceutical dosage form is in an amount of from 250 mg to 700 mg calculated as saquinavir free base.

5. The dosage form according to claims 1 to 4, wherein the saquinavir mesylate in the pharmaceutical dosage form is in an amount of 500 mg calculated as saquinavir free base.

6. The dosage form according to claims 1 to 5, wherein lactose monohydrate has a particle size of from 30 micrometer to 200 micrometer.

7. The dosage form according to claim 6, wherein the lactose monohydrate has a particle size of from 100 micrometer to 150 micrometer.

8. The dosage form according to claims 1 to 7, further comprising microcrystalline cellulose.

9. The dosage form according to claim 8, wherein the microcrystalline cellulose is present from 5% to 20% of the kernel weight.

10. The dosage form according to claims 1 to 9, wherein the dosage form further comprises a lubricant.

11. The dosage form according to claim 10, wherein the lubricant is magnesium stearate.

12. The dosage form according to claims 1 to 11, wherein the kernel of the pharmaceutical dosage form consists of an admixture of a granulation and an extra-granulation, wherein said granulation comprises the micronized saquinavir mesylate, the binder, the carrier, and the disintegrant, and the extra-granulation comprises disintegrant, wherein the ratio of disintegrant in the granulation to disintegrant in the extra-granulation is from 3:1 to 1:1.

13. The dosage form according to claim 12, further comprising a lubricant, which is comprised in the extra-granulation of the kernel.

14. The dosage form according to claims 1 to 13, which is selected from the group consisting of a tablet, a capsule and a caplet.

15. The dosage form according to claims 1 to 14, having a weight of from 400 mg to 1.5 g.

16. The solid unit oral pharmaceutical dosage form according to claims 1 to 15, wherein the saquinavir mesylate is in crystalline form.

17. A process for making a solid unit oral pharmaceutical dosage form according to claims 1 to 16, comprising micro-granulating a blend of micronized saquinavir mesylate, polyvinylpyrrolidone, lactose monohydrate, and of a pharmaceutically active disintegrant and comprising subsequent milling.

18. A process according to claim 17, comprising the steps of:
a) blending micronized saquinavir mesylate in an amount of from 200 mg to 800 mg (calculated as free base) per unit dosage with a hydrophilic carrier and disintegrant in a high shear granulator,
b) spraying or slowly adding an aqueous binder solution in water to the powder mix from step (a) while mixing to achieve the optimal granulation end point,
c) de-lumping the wet granulation from step (b) into a uniform granulation,
d) drying the wet granulation from step (c) in a forced-air oven set at 40° to 50°C or in a fluid bed dryer with inlet air temperature of 50° to 60°C until the moisture content of the granulation is in the range of 1.5-2%,
e) milling the dried granulation from step (d),
f) blending the milled granulation from step (e) with a suitable tablet diluent,
g) lubricating the blend from step (f) with a suitable lubricant,
h) compressing the final blend from step (g) on a tablet press and
i) aqueous film coating the tablet from step (h).

19. The solid unit oral pharmaceutical dosage form according to claims 1 to 16 for use in therapy.

20. The solid unit oral pharmaceutical dosage form according to claims 1 to 16 for use in therapy of HIV mediated diseases.

21. Use of a solid unit oral pharmaceutical dosage form according the claims 1 to 16 for the manufacture of a medicament for the treatment of a HIV mediated disease.

## Patentansprüche

1. Feste orale pharmazeutische Dosiereinheitsform von Saquinavir-Mesylat, umfassend 60 % bis 80 % mikronisiertes Saquinavir-Mesylat, bezogen auf das Mesylatsalz, 4 % bis 8 % Polyvinylpyrrolidon, 3 % bis 10 % eines pharmazeutisch unbedenklichen Sprengmittels, ausgewählt aus Croscarmellose-Natrium und Crospovidon, und 3 % bis 10 % Lactose-Monohydrat, wobei sich jeder Prozentsatz auf das Kerngewicht der pharmazeutischen Dosierform bezieht.

2. Dosierform nach Anspruch 1, wobei das Saquinavir-Mesylat 70 % bis 75 % des Kerngewichts der pharmazeutischen Dosierform beträgt.

3. Dosierform nach Anspruch 1 oder 2, wobei das Saquinavir-Mesylat in der pharmazeutischen Dosierform in einer Menge von 200 mg bis 700 mg, berechnet als freie Saquinavir-Base, vorhanden ist.

4. Dosierform nach den Ansprüchen 1 bis 3, wobei das Saquinavir-Mesylat in der pharmazeutischen Dosierform in einer Menge von 250 mg bis 700 mg, berechnet als freie Saquinavir-Base, vorhanden ist.

5. Dosierform nach den Ansprüchen 1 bis 4, wobei das Saquinavir-Mesylat in der pharmazeutischen Dosierform in einer Menge von 500 mg, berechnet als freie Saquinavir-Base, vorhanden ist.

6. Dosierform nach den Ansprüchen 1 bis 5, wobei das Lactose-Monohydrat eine Partikelgröße von 30 Mikrometer bis 200 Mikrometer aufweist.

7. Dosierform nach Anspruch 6, wobei das Lactose-Monohydrat eine Partikelgröße von 100 Mikrometer bis 150 Mikrometer aufweist.

8. Dosierform nach den Ansprüchen 1 bis 7, ferner umfassend mikrokristalline Cellulose.

9. Dosierform nach Anspruch 8, wobei die mikrokristalline Cellulose in 5 % bis 20 % des Kerngewichts vorhanden ist.

10. Dosierform nach den Ansprüchen 1 bis 9, wobei die Dosierform ferner ein Gleitmittel umfasst.

11. Dosierform nach Anspruch 10, wobei das Gleitmittel Magnesiumstearat ist.

12. Dosierform nach den Ansprüchen 1 bis 11, wobei der Kern der pharmazeutischen Dosierform aus einer Beimischung aus einem Granulat und einem Extra-Granulat besteht, wobei das Granulat das mikronisierte Saquinavir-Mesylat, das Bindemittel, den Träger und das Sprengmittel umfasst, und das Extra-Granulat Sprengmittel umfasst, wobei das Verhältnis des Sprengmittels in dem Granulat zum Sprengmittel in dem Extra-Granulat 3:1 bis 1:1 beträgt.

13. Dosierform nach Anspruch 12, ferner umfassend ein Gleitmittel, das in dem Extra-Granulat des Kerns enthalten ist.

14. Dosierform nach den Ansprüchen 1 bis 13, die ausgewählt ist aus der Gruppe bestehend aus einer Tablette, einer Kapsel und einer kapselförmigen Tablette.

15. Dosierform nach den Ansprüchen 1 bis 14, die ein Gewicht von 400 mg bis 1,5 g aufweist.

16. Feste orale pharmazeutische Dosiereinheitsform nach den Ansprüchen 1 bis 15, wobei das Saquinavir-Mesylat in kristalliner Form vorliegt.

17. Verfahren zur Herstellung einer festen oralen pharmazeutischen Dosiereinheitsform nach den Ansprüchen 1 bis 16, umfassend das Mikrogranulieren einer Mischung aus mikronisiertem Saquinavir-Mesylat, Polyvinylpyrrolidon, Lactose-Monohydrat und einem pharmazeutisch aktiven Sprengmittel und umfassend das anschließende Mahlen.

18. Verfahren nach Anspruch 17, umfassend die folgenden Schritte:
a) Vermischen des mikronisierten Saquinavir-Mesylats in einer Menge von 200 mg bis 800 mg (berechnet als freie Base) pro Dosiereinheit mit einem hydrophilen Träger und einem Sprengmittel in einem Granulator mit hoher Scherwirkung,
b) Sprühen oder langsames Zugeben einer wässrigen Bindemittellösung in Wasser zu der Pulvermischung aus Schritt (a) während des Mischens, um den optimalen Granulationsendpunkt zu erreichen,
c) Entklumpen des Feuchtgranulats aus Schritt (b) zu einem gleichmäßigen Granulat,
d) Trocknen des Feuchtgranulats aus Schritt (c) in einem Druckluftofen, der auf 40 ° bis 50 °C eingestellt ist, oder in einem Wirbelschichttrockner mit einer Einlasstemperatur von 50 ° bis 60 °C, bis der Feuchtigkeitsgehalt des Granulats in einem Bereich von 1,5-2 % liegt,
e) Mahlen des getrockneten Granulats aus Schritt (d),
f) Mischen des gemahlenen Granulats aus Schritt (e) mit einem geeigneten Tablettenverdünnungsmittel,
g) Schmieren der Mischung aus Schritt (f) mit einem geeigneten Gleitmittel,
h) Komprimieren der endgültigen Mischung aus Schritt (g) auf einer Tablettenpresse und
i) wässriges Filmbeschichten der Tablette aus Schritt (h).

19. Feste orale pharmazeutische Dosiereinheitsform nach den Ansprüchen 1 bis 16 zur Verwendung bei der Therapie.

20. Feste orale pharmazeutische Dosiereinheitsform nach den Ansprüchen 1 bis 16 zur Verwendung bei der Therapie von HIV-vermittelten Krankheiten.

21. Verwendung einer festen oralen pharmazeutischen Dosiereinheitsform nach den Ansprüchen 1 bis 16 zur Herstellung eines Medikaments zur Behandlung einer HIV-vermittelten Krankheit.

## Revendications

1. Forme dosifiée pharmaceutique orale unitaire solide de mésylate de saquinavir comprenant de 60 % à 80 % de mésylate de saquinavir micronisé sur la base du sel de mésylate, de 4 % à 8 % de polyvinylpyrrolidone, de 3 % à 10 % d'un agent désintégrant pharmaceutiquement acceptable choisi parmi la croscarmellose sodique et la crospovidone et de 3 % à 10 % de lactose monohydraté, dans laquelle chaque pourcentage correspond au poids du noyau de la forme dosifiée pharmaceutique.

2. Forme dosifiée selon la revendication 1, dans laquelle le mésylate de saquinavir représente de 70 % à 75 % du poids du noyau de la forme dosifiée pharmaceutique.

3. Forme dosifiée selon la revendication 1 ou 2, dans laquelle le mésylate de saquinavir dans la forme dosifiée pharmaceutique est présent en une quantité de 200 mg à 700 mg calculée en tant que base libre du saquinavir.

4. Forme dosifiée selon les revendications 1 à 3, dans laquelle le mésylate de saquinavir dans la forme dosifiée pharmaceutique est présent en une quantité de 250 mg à 700 mg calculée en tant que base libre du saquinavir.

5. Forme dosifiée selon les revendications 1 à 4, dans laquelle le mésylate de saquinavir dans la forme dosifiée pharmaceutique est présent en une quantité de 500 mg calculée en tant que base libre du saquinavir.

6. Forme dosifiée selon les revendications 1 à 5, dans laquelle le lactose monohydraté a une taille de particules de 30 micromètres à 200 micromètres.

7. Forme dosifiée selon la revendication 6, dans laquelle le lactose monohydraté a une taille de particules de 100 micromètres à 150 micromètres.

8. Forme dosifiée selon les revendications 1 à 7, comprenant en outre de la cellulose microcristalline.

9. Forme dosifiée selon la revendication 8, dans laquelle la cellulose microcristalline est présente à hauteur de 5 % à 20 % du poids du noyau.

10. Forme dosifiée selon les revendications 1 à 9, dans laquelle la forme dosifiée comprend en outre un lubrifiant.

11. Forme dosifiée selon la revendication 10, dans laquelle le lubrifiant est du stéarate de magnésium.

12. Forme dosifiée selon les revendications 1 à 11, dans laquelle le noyau de la forme dosifiée pharmaceutique consiste en un mélange d'une granulation et d'une extra-granulation, ladite granulation comprenant le mésylate de saquinavir micronisé, le liant, l'excipient et l'agent désintégrant, et l'extra-granulation comprenant un agent désintégrant, le rapport de l'agent désintégrant dans la granulation à l'agent désintégrant dans l'extra-granulation étant de 3/1 à 1/1.

13. Forme dosifiée selon la revendication 12, comprenant en outre un lubrifiant qui est compris dans l'extra-granulation du noyau.

14. Forme dosifiée selon les revendications 1 à 13, qui est choisie dans le groupe constitué d'un comprimé, d'une gélule et d'un comprimé-capsule.

15. Forme dosifiée selon les revendications 1 à 14, ayant un poids de 400 mg à 1,5 g.

16. Forme dosifiée pharmaceutique orale unitaire solide selon les revendications 1 à 15, dans laquelle le mésylate de saquinavir est sous forme cristalline.

17. Procédé de fabrication d'une forme dosifiée pharmaceutique orale unitaire solide selon les revendications 1 à 16, comprenant la micro granulation d'un mélange de mésylate de saquinavir micronisé, de polyvinylpyrrolidone, de lactose monohydraté et d'un agent désintégrant pharmaceutiquement actif et comprenant ensuite le broyage.

18. Procédé selon la revendication 17, comprenant les étapes de :
a) mélange de mésylate de saquinavir micronisé en une quantité de 200 mg à 800 mg (calculée en tant que base libre) par dose unitaire avec un excipient hydrophile et un agent désintégrant dans un granulateur à fort cisaillement,
b) pulvérisation ou d'ajout lent d'une solution aqueuse liante dans de l'eau au mélange en poudre de l'étape (a) tout en mélangeant pour atteindre le point final optimal de granulation,
c) désagrégation de la granulation humide de l'étape (b) en une granulation uniforme,
d) séchage de la granulation humide de l'étape (c) dans un four à air pulsé réglé à 40 °C-50 °C ou dans un séchoir à lit fluidisé avec une température d'air d'entrée de 50 °C à 60 °C jusqu'à ce que la teneur en humidité de la granulation soit dans la plage de 1,5 à 2 %,
e) broyage de la granulation séchée de l'étape (d),
f) mélange de la granulation broyée de l'étape (e) avec un diluant pour comprimés approprié,
g) lubrification du mélange de l'étape (f) avec un lubrifiant approprié,
h) compression du mélange final de l'étape (g) sur une presse à comprimés et
i) enrobage du comprimé de l'étape (h) avec une pellicule aqueuse.

19. Forme dosifiée pharmaceutique orale unitaire solide selon les revendications 1 à 16 pour une utilisation dans un traitement.

20. Forme dosifiée pharmaceutique orale unitaire solide selon les revendications 1 à 16 pour une utilisation dans le traitement des maladies médiées par le VIH.

21. Utilisation d'une forme dosifiée pharmaceutique orale unitaire solide selon les revendications 1 à 16 pour la fabrication d'un médicament pour une utilisation dans le traitement d'une maladie médiée par le VIH.
